# EUROPEAN PATENT SPECIFICATION

(11) **EP 3 876 713 B1**
(45) Date of publication and mention of the grant of the patent: **02.10.2024**
(21) Application number: 19798323.2
(22) Date of filing: 11.11.2019
(51) Int. Cl.: A01K 67/027, C07K 14/47

(54) **PATIENT-DERIVED AMYLOID XENOGRAFT RODENT MODEL**
VOM PATIENTEN GEWONNENES AMYLOID-XENOTRANSPLANTAT-NAGETIERMODELL
MODÈLE DE RONGEUR DE XÉNOGREFFE AMYLOÏDE DÉRIVÉE D'UN PATIENT

(30) Priority: 09.11.2018 EP 18205502
(43) Date of publication of application: 15.09.2021
(73) Proprietor: Neurimmune AG, 8952 Schlieren (CH)
(72) Inventor: MICHALON, Aubin, 5400 Baden (CH); GRIMM, Jan, 8600 Dübendorf (CH)
(74) Representative: Jaekel, Robert Sebastian
(86) International application number: PCT/EP2019/080829
(87) International publication number: WO 2020/094883

(56) References cited:
- WO-A1-2016/120811
- I SLAMOVA: "Modelling amyloidosis in mice", 2016, XP055574169, Retrieved from the Internet <URL:http://discovery.ucl.ac.uk/1534595/1/PhD%20Thesis%20-%20Ivana%20Slamova%20-%202016.pdf> [retrieved on 20190326]
- MAMBULE C ET AL: "Enhancement of AA-amyloid formation in mice by transthyretin amyloid fragments and polyethylene glycol", BIOCHIMICA ET BIOPHYSICA ACTA (BBA) - GENERAL SUBJECTS, ELSEVIER, AMSTERDAM, NL, vol. 1474, no. 3, May 2000 (2000-05-01), pages 331 - 336, XP004276572, ISSN: 0304-4165, DOI: 10.1016/S0304-4165(00)00032-5
- YOHEI MISUMI ET AL: "Fibroblasts endocytose and degrade transthyretin aggregates in transthyretin-related amyloidosis", LABORATORY INVESTIGATION, vol. 93, no. 8, August 2013 (2013-08-01), The United States and Canadian Academy of Pathology, Inc., pages 911 - 920, XP055657289, ISSN: 0023-6837, DOI: 10.1038/labinvest.2013.83
- JONATHAN S WALL ET AL: "Development and evaluation of agents for targeting visceral amyloid", TIJDSCHRIFT VOOR NU[C]LEAIRE GENEESKUNDE, vol. 33, no. 4, December 2011 (2011-12-01), Netherlands, pages 807, XP055574438, ISSN: 1381-4842
- LORENA SAELICES ET AL: "Amyloid seeding of transthyretin by ex vivo cardiac fibrils and its inhibition", PROCEEDINGS OF THE NATIONAL ACADEMY OF SCIENCES OF THE UNITED STATES OF AMERICA, vol. 115, no. 29, 28 June 2018 (2018-06-28), US, pages E6741 - E6750, XP055574643, ISSN: 0027-8424, DOI: 10.1073/pnas.1805131115

## Description

### FIELD OF THE INVENTION

The present invention relates to the field of *in vivo* animal models and specifically to a patient-derived amyloid xenograft (PDAX) rodent model, uses thereof and methods of producing the PDAX model. The present invention also relates to methods and processes to determine/obtain, characterize, validate, develop, and/or quality control anti-amyloid drugs suitable for the treatment of an amyloidosis or amyloid-related disease.

### BACKGROUND OF THE INVENTION

Amyloidoses are protein-folding diseases characterized by extracellular deposition of a specific soluble precursor protein that aggregates in the form of insoluble fibrils; see Hazenberg, Rheum. Dis. Clin. North Am. 39 (2013), 323-345 for review. These rigid and unbranching fibrils, approximately 10 nm in diameter, are characterized by a molecular β-pleated sheet structure that is usually composed of peptides arranged in an antiparallel configuration. This structure of the fibrils is responsible for its insolubility, resistance to proteolysis, and binding affinity for the amyloid-specific Congo red and thioflavine-S dyes. Three mechanisms seem to operate independently or in combination: the precursor protein may have an intrinsic propensity to misfold that becomes evident with aging (wild-type transthyretin) or at high serum levels (serum amyloid A protein and immunoglobulin free light chains); a hereditary acquired mutated protein (transthyretin); and proteolytic remodelling of the precursor protein (β-amyloid precursor protein). Interaction with the extracellular matrix also seems to be important and may be related to preferential deposition of amyloid in some organs or tissues. Extracellular deposition of amyloid fibrils in organs and tissues results in tissue infiltration and swelling leading to progressive loss off function of the affected organ.

Amyloidosis are roughly distinguished in localized forms where deposits are restricted to one organ or site of the body like Alzheimer's disease with amyloid β protein plaques in the brain or diabetes mellitus type 2 with amyloid amylin deposition in the islands of Langerhans in the pancreas, and systemic forms where fibril deposition occurs in various organs and tissues throughout the body. One of the most frequent systemic amyloidoses is amyloid transthyretin (ATTR) amyloidosis where ATTR fibrils accumulate in multiple organs and tissues and lead to multiple organ dysfunctions with rapid disease progression and fatal consequences.

ATTR amyloidosis shows two main forms of clinical presentation, with predominant amyloid fibril accumulation in cardiac tissues leading to cardiomyopathy (ATTR-CM), and fibril accumulation in nerve fibres leading to polyneuropathy (ATTR-PN) (Ando et al., Orphanet J. Rare Dis. 8:31 (2013) 1-18). TTR has an innate ability to aggregate into insoluble amyloid fibres. Amyloid misfolding occurs spontaneously at a low rate in putatively all individuals and increases with aging and senescence. It is hypothesized that in healthy subjects the immune system has the capacity to eliminate the amyloid fibrils; however, for reasons not fully elucidated yet, amyloid fibrils escape immune surveillance and accumulate up to toxic levels in ATTR patients. Regarding ATTR-CM, there is no specific treatment available yet. Usual heart failure treatments such as beta-blockers, angiotensin-converting enzyme inhibitors, and angiotensin receptor blockers are poorly tolerated in ATTR-CM and should be avoided (Gertz et al, J. Am. Coll. Cardiol. 66 (2015), 2451-2466). In absence of treatment able to specifically decrease the amount of ATTR amyloid, heart transplantation is still the only available approach to restore cardiac function but with poor applicability to the elderly and fragile patient population. Liver transplantation was considered an option for mutant ATTR, but both peripheral neuropathy as well as cardiac amyloidosis can progress after liver transplantation. Tafamidis (Vyndaqel^{®}), a transthyretin stabilizing drug, was approved in Europe in November 2011 for patients with early stage ATTR-PN. However, in a post-approval trail, neurological impairment scores worsened by 55% after 1 year of tafamidis treatment, suggesting that it could not stop disease progression (Gertz et al, J. Am. Coll. Cardiol. 66 (2015), 2451-2466). Similar results were obtained recently with tafamidis in ATTR-CM patients. Tafamidis reduced the speed of disease progression leading to 35% better survival and hospitalization rate after 30 months of treatment in ATTR-CM patients (Maurer et al., N. Engl. J. Med. 379 (2018), 1007-1016); however, similar to what has been observed in ATTR-PN, tafamidis treatment was more beneficial to early stage patients and reduced but did not stop disease progression, and did not provide symptom regression to patients.

In the development of new drug therapies, animal models are essential in order to assess a drug's efficiency and safety. Amyloidoses drug treatments, not only in ATTR but in most if not all amyloidoses, have so far been studied using transgenic animal models, and especially transgenic mouse models, which mostly rely on mutations in respective precursor proteins observed in human patients. For example, multiple transgenic mouse models have been presented to the scientific community as attempts to model ATTR amyloidosis, but they all suffer from major limitations including absence or rarity of ATTR amyloid deposits, high variability depending on age, gender, genetic background and housing conditions, and in all cases, absence of phenotypes mimicking patient symptoms (Takaoka et al., Transgenic Research 6 (1997), 261-269; Teng et al., Lab Invest. 81 (2001), 385-396; Sousa et al., Am. J. Path. 161 (2002), 1935-1948; Noguchi, Exp. Anim. 51 (2002), 309-316; Panayiotou, BB reports 8 (2016), 48-54).

Analyses characterizing the transgenic mouse models indicate that these transgenic mouse models present non-amyloid TTR deposition, which does not have the characteristic tinctorial properties of amyloid, is soluble instead of being insoluble, and does not induce toxicity in mice. Accordingly, the models available are not appropriate for assessing a compound's potential to bind and clear insoluble amyloid fibrils in these models which present soluble deposits only. Accordingly, there is a need for new systems that appropriately model amyloidoses in order to enable the development of new efficient amyloidosis therapies.

### SUMMARY OF THE INVENTION

The present invention generally relates to a patient-derived amyloid xenograft (PDAX) rodent model as characterized in the claims. In accordance with the present invention, the animal model is characterized by an implant of amyloid transthyretin (ATTR) fibrils derived from the tissue or organ of a patient suffering from an ATTR amyloidosis.

In accordance with the present invention, the tissue is cardiac tissue, kidney tissue, liver tissue, gastro-intestinal tissue, skin tissue, muscle tissue, tongue tissue, fat tissue, salivary gland tissue, lymph node tissue, brain tissue, pancreatic tissue or any ATTR amyloidoma, wherein the amyloid fibrils are subcutaneously implanted, or implanted in the kidneys or subcapsular, in the peritoneum, the muscles, the brain, the ventricles, the nerves, the eyes, the tongue or the heart of the animal model.

In accordance with the present invention, the animal is a rodent, for example a mouse or rat, *i.e.* the non-human animal model is a rodent and is non-transgenic, at least for the amyloid fibril protein.

In another aspect, the present invention relates to a method of determining and/or obtaining an anti-amyloid drug suitable for the treatment of an amyloidosis or amyloid-related disease comprising administering the drug to the model of the present invention; and determining amyloid fibrils in the model, wherein the accelerated elimination or reduction of the amyloid fibrils upon administration of the drug compared to a control is indicative for the suitability for the anti-amyloid drug, wherein the drug comprises an anti-amyloid fibril protein antibody. In one embodiment, the method comprises the collection of tissue biopsies at a first and second time point after administration, and analysis and quantification of amyloid fibrils including immunohistochemistry. Preferably, the amount of amyloid fibrils is expressed as percentage of the implant tissue area, and the amyloid staining area covering the implant tissue area in the group treated with the drug is significantly lower than in the control group. In an additional or alternative embodiment, the accelerated elimination or reduction of the amyloid fibrils is observed in a dose dependent manner.

In accordance with the method of the present invention, the drug comprises an anti-amyloid fibril protein antibody.

In another embodiment of the present invention, the control is a corresponding isotype antibody.

In a further embodiment of the present invention, the antibody is a human antibody or a human chimeric antibody comprising a rodent constant domain from the same species as the animal employed in the model.

In a further embodiment of the present invention, the drug is administered intravenously, intraperitoneally, subcutaneously or orally.

A further aspect of the present invention relates to a method for characterization, validation, development and/or quality control of an anti-amyloid drug suitable for the treatment of an amyloidosis or amyloid-related disease comprising subjecting the drug to the method according to the present invention for determining and/or obtaining an anti-amyloid drug; communicating the information obtained by the method to a client, contracting party or cooperation partner and/or selecting the drug that has been determined to be a suitable anti-amyloid drug;. Thus, one aspect of the present invention relates to the use of the model according to the present invention for drug characterization, quality control and/or development, pre- and/or co-clinical trials or selecting or validating a drug in the manufacture of a medicament for the treatment of an amyloidosis or an amyloid-related disease.

In another aspect, the present invention relates to a method of producing a patient-derived amyloid xenograft (PDAX) rodent model of the present invention comprising isolation of ATTR fibrils from a tissue biopsy obtained from a patient suffering from an ATTR amyloidosis, and implantation of the isolated amyloid fibrils in a rodent, preferably wherein the total protein concentration is about 0.5 to 5 mg/ml, preferably about 1 to 4 mg/ml and most preferably about 2 ± 0.5 mg/ml, preferably as determined with the BCA assay.

### BRIEF DESCRITION OF THE DRAWINGS

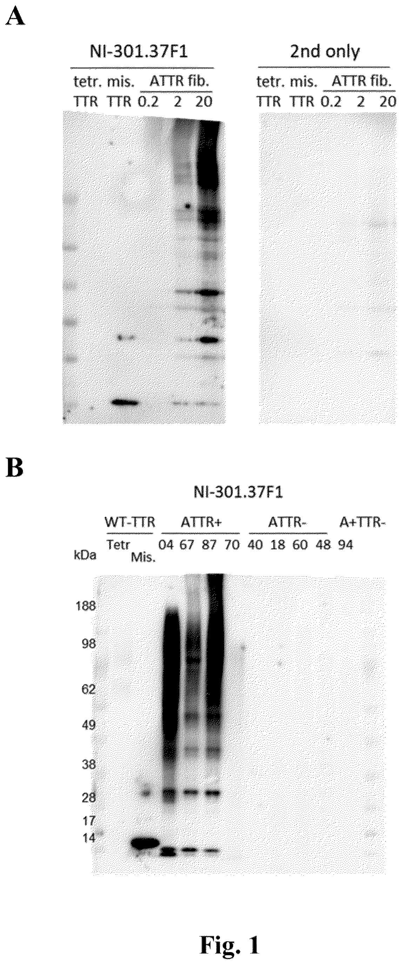

- **Fig. 1:**: Presence of large ATTR aggregates detected by NI-301.37F1 in amyloid fibril extracts prepared from human heart tissues. **A** Semi-native SDS-PAGE and Western blot analysis with NI-301.37F1 of tetrameric WT-TTR (100 ng, not detected), misfolded WT-TTR (100 ng), and 0.2, 2.0 and 20 µg per lane of ATTR fibril extract. **B** Western blot with antibody NI-301.37F1 (10nM) binding to ATTR fibrils extracted from post-mortem cardiac tissues obtained from 4 different patients with ATTR amyloidosis (sample numbers 4, 67, 87 and 70) with 30 s exposure time. Binding to sample 70 is very faint and consistent with the very low amount of ATTR detect by IHC in this tissue. NI-301.37F1 did not bind to the same protein fraction prepared from human heart tissues without ATTR amyloidosis (samples 40, 18, 60 and 48), and from one sample with amyloidosis without TTR (A+TTR- sample 94).

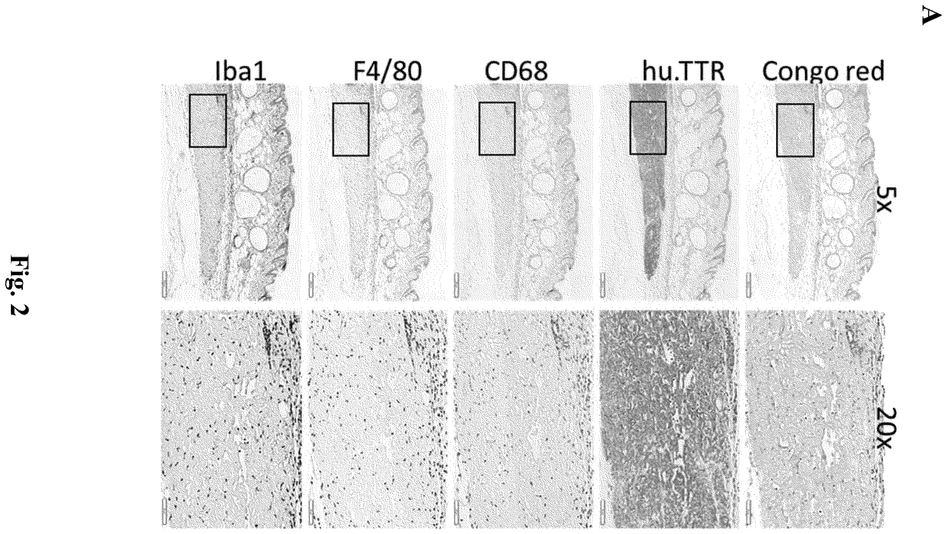

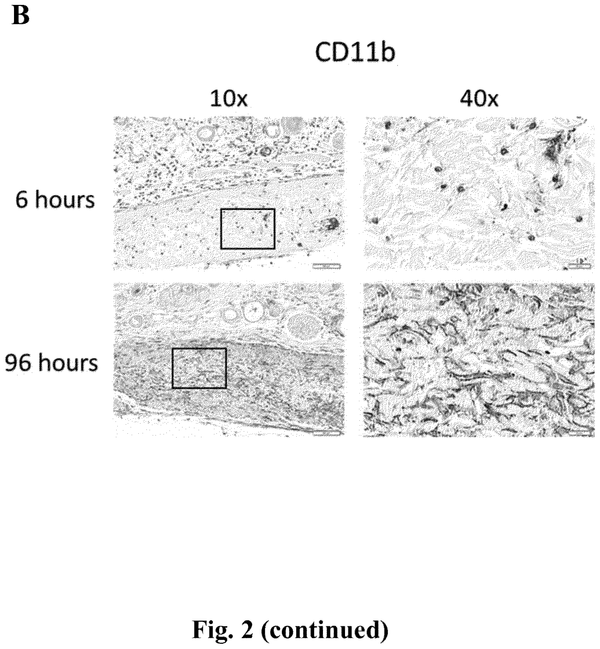

- **Fig. 2:**: Characterization of subcutaneous ATTR fibril implants in mice. **A** 24 hours after implantation, fibril grafts were recognizable by their characteristic shape and morphology different from neighbouring skin tissue. Fibril grafts were Congo red positive, and stained strongly by IHC with the human TTR antibody Dako A0002, indicating presence of ATTR fibrils. The fibril grafts were infiltrated with cells which were negative for the usual murine macrophage markers CD68, F4/80 and Iba1. **B** Fibril implants stained positive by IHC with CD11b antibody indicating that the cells infiltrating the ATTR implant and removing fibrils are neutrophils.

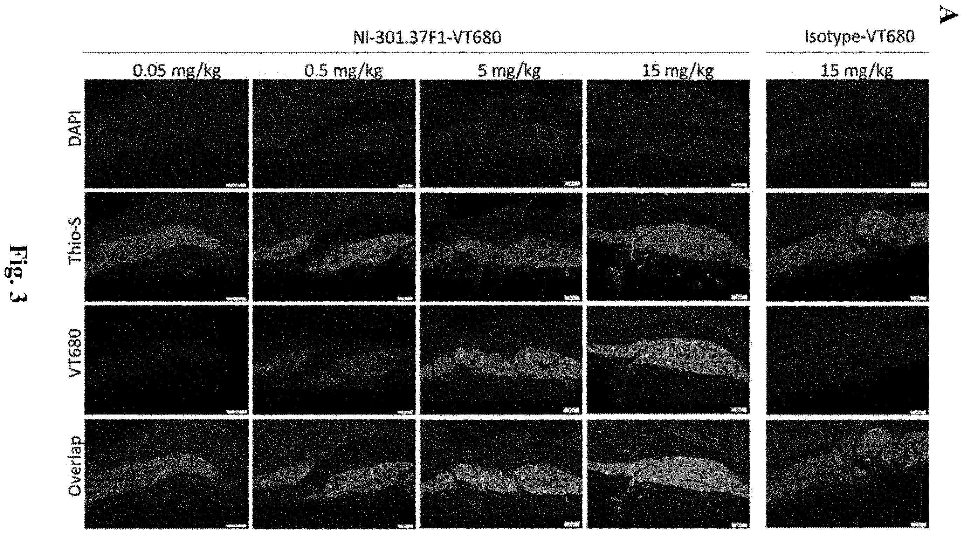

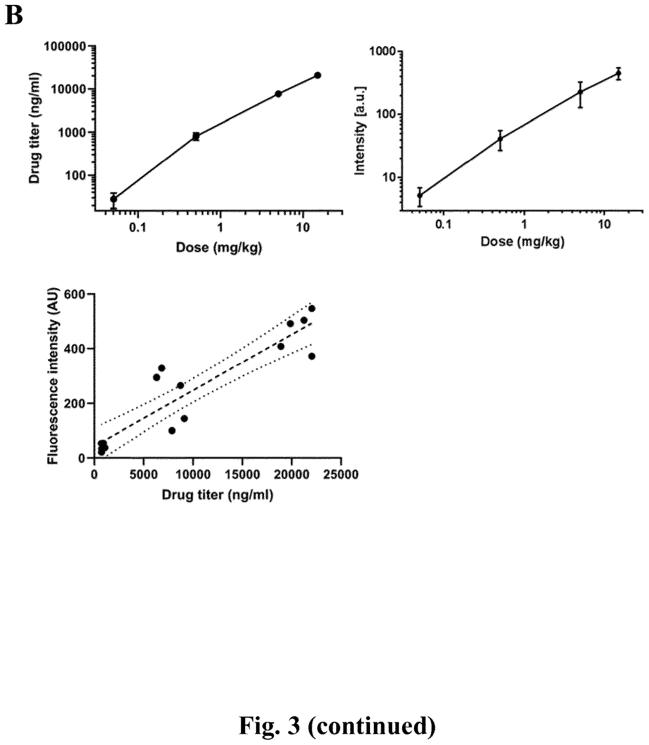

- **Fig. 3:**: The ATTR-specific antibody NI-301.37F1 binds patient-derived ATTR fibrils in a dose-dependent manner *in vivo.* **A** 48 hours after fibril implantation s.c. and antibody injection i.v., fluorescently-labelled NI-301.37F1 (NI-301.37F1-VT680) accumulated in a dose-dependent manner on the ATTR fibril implants detected by thioflavine-S fluorescence. This was not observed with the isotype antibody (Isotype-VT680). Representative images, with n=5 mice per dose level. **B** Linear correlation was observed between antibody concentration in plasma and antibody density on ATTR fibril graft *in vivo.* Dose-dependent increases on NI-301.37F1-VT680 concentration in plasma (upper left panel) and NI-301.37F1-VT680 fluorescence intensity at the fibril craft (upper right panel) across the dose range 0.05-15 mg/kg have been shown. Linear correlation between NI-301.37F1-VT680 concentrations and fluorescence intensity across the dose range 0.5-15 mg/kg (lower panel). Dotted line: linear fit ±95%CI, R²=0.846, n=5 per dose level.

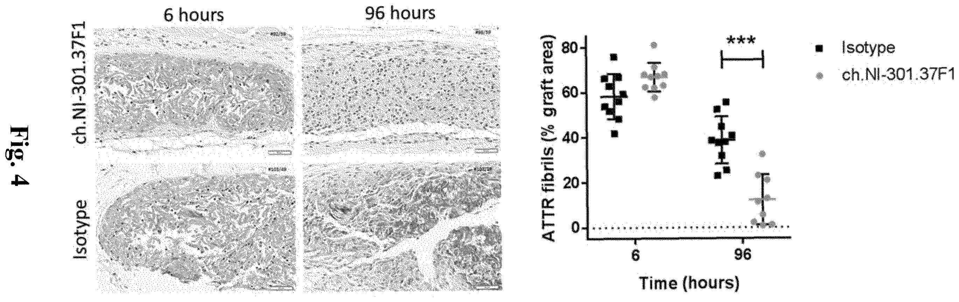

- **Fig. 4:**: Treatment with mouse chimeric NI-301.37F1 accelerates ATTR fibril clearance *in vivo.* Quantification by IHC of human TTR remaining in ATTR fibril implants 6 and 96 hours after grafting and treatment administration. Administration of ch.NI-301.37F1 at 5 mg/kg i.v. accelerated fibril elimination. n=9-10 mice per group, 2-way ANOVA with Sidak's multiple comparison test: p<0.0001.

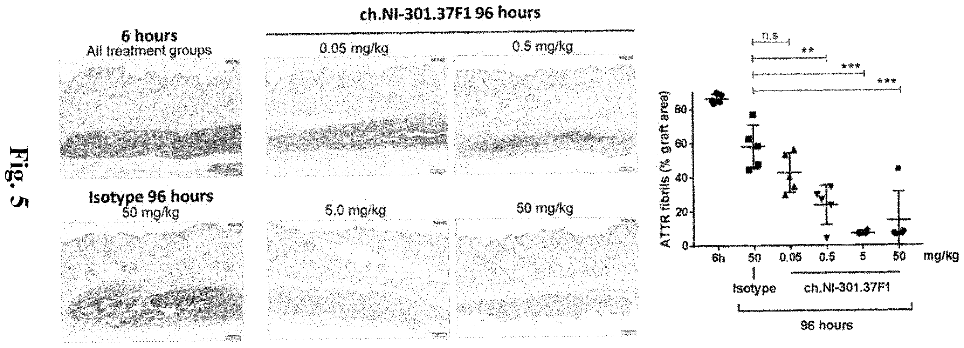

- **Fig. 5:**: Treatment with mouse chimeric NI-301.37F1 accelerates *in vivo* ATTR fibril clearance in a dose-dependent manner. ATTR fibrils quantification using TTR IHC. N=5 per group, 1-way ANOVA with Dunnett's multiple comparison test: n.s.: not significant (p>0.05), **: p=0.001, ***: p<0.001.

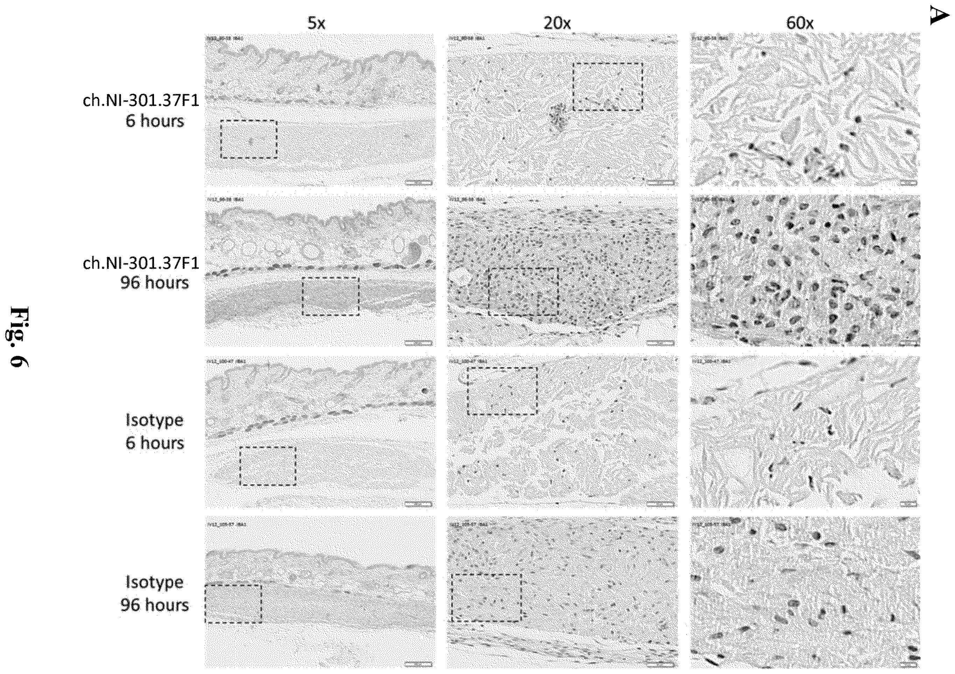

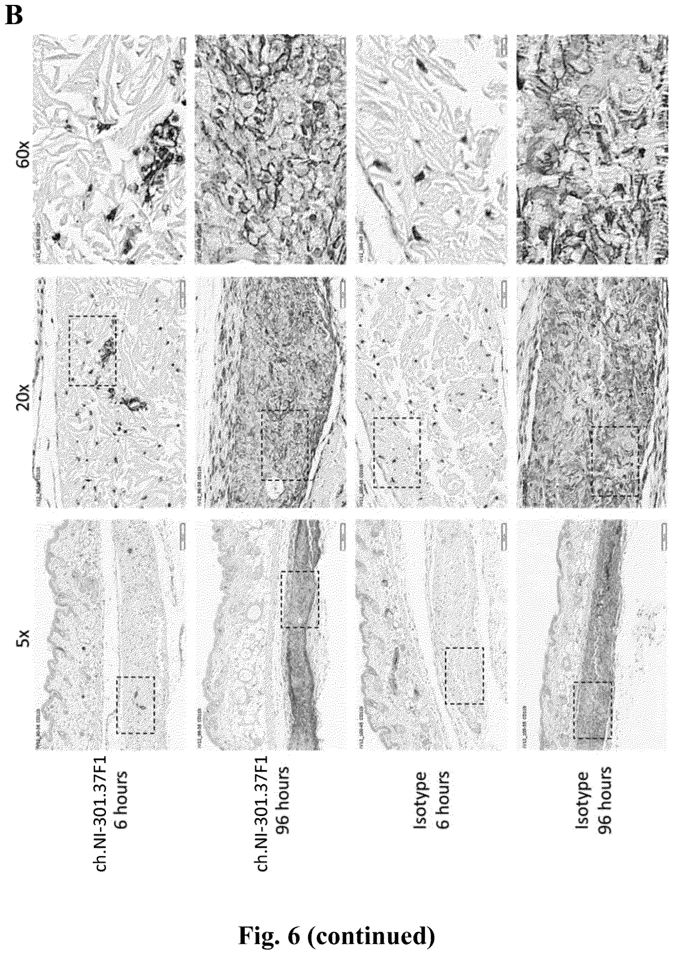

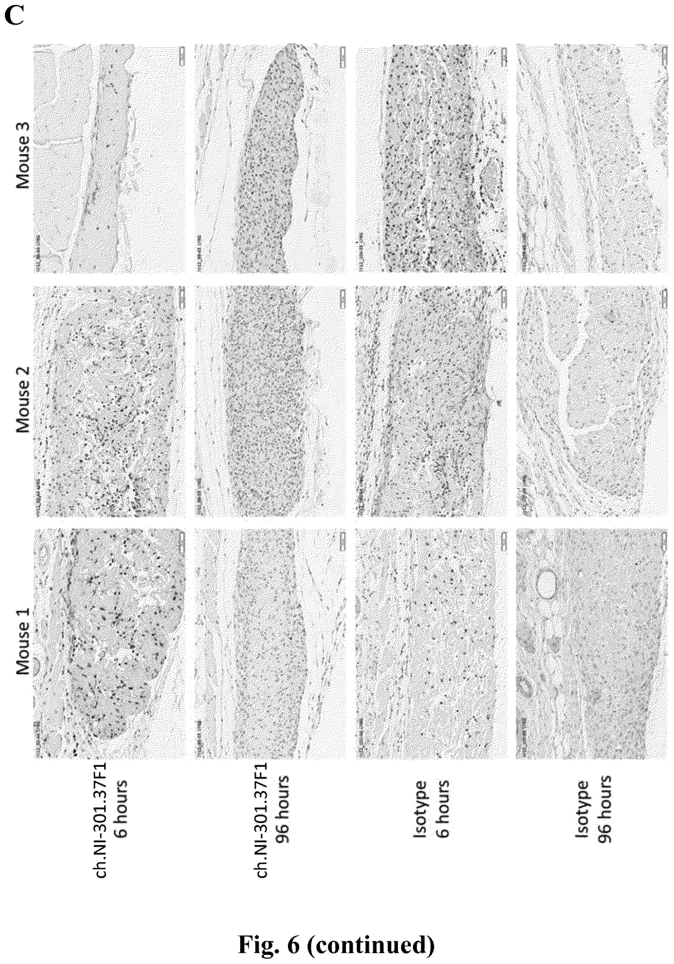

- **Fig. 6:**: Characterization of subcutaneous ATTR fibril implants in mice upon treatment with mouse chimeric NI-301.37F 1. A IBA1 staining on subcutaneous ATTR fibril grafts, 6 and 96 hours after grafting and treatment with ch.NI-301.301.37F1 or isotype at 5 mg/kg i.v. Representative images from n=5 mice per treatment group and time point, with 5 non-consecutive sections per animal. **B** CD11B staining on ATTR fibril grafts, 6 and 96 hours after grafting and treatment with ch.NI-301.37F1 or isotype at 5 mg/kg i.v. Representative images from n=5 mice per treatment group and time point, with 5 non-consecutive sections per animal. **C** LY6G staining on ATTR fibril grafts, 6 and 96 hours after grafting and treatment with ch.NI-301.37F1 or isotype at 5 mg/kg i.v. Representative images from 3 different mice per treatment group and time point. Staining performed on 5 mice per group and 2 non-consecutive sections per animal.

### DETAILED DESCRITION OF THE INVENTION

The present invention relates to a patient-derived amyloid xenograft (PDAX) rodent model as characterized in the claims. In the absence of a suitable *in vivo* animal model for testing anti-amyloid drugs, the inventors have developed a simple but highly efficient model for this purpose which advantageously is not necessarily based on transgenic animals. The PDAX model is characterized by an implant of amyloid transthyretin (ATTR) fibrils isolated from the tissue or organ of a patient suffering from an ATTR amyloidosis. Surprisingly, it has been found that the amyloid fibril implant is maintained in the animal for several days and is even accessible to drug treatment. In particular, as shown exemplary for a mouse implanted with ATTR fibrils in Examples 1 and 2, the amyloid fibril implant is tolerated in the laboratory animal and presents the characteristic amyloid features in tissue biopsies following histological analyses. Furthermore, as shown in Examples 3 and 4, the PDAX model has been validated with an anti-ATTR specific antibody that has been shown to actively promote clearing of ATTR fibrils *in vivo* in another mouse model. The PDAX model is therefore highly suitable for testing compounds or drugs for their suitability as anti-amyloid drugs.

Previously, Higaki et al. in a poster presentation at the XVIth International Symposium on Amyloidosis; March 26-29, 2018; Kumamoto, Japan, reported on an *in vivo* model of target engagement and antibody-dependent cellular phagocytosis (ADCP) by a systemically administered, humanized antibody against misfolded TTR (mis-TTR antibody), wherein aggregates of His-tagged TTR-V30M suspended in basement membrane matrix (Matrigel) are implanted subcutaneously into mice.

However, while in principle the method of implantation could be used for generating a PDAX animal model in accordance with the present invention, the ADCP model mice of Higaki *et al. per se* does represent an appropriate animal model according the present invention and may not be suitable for the intended purpose.

For example, despite the fact that human ATTR amyloidosis is a monogenetic disease, there are large variations in its phenotype and ATTR fibril composition, for example distinct types of amyloid fibrils, type A consisting of C-terminal ATTR fragments and full-length TTR, whereas the other, type B, consists only of full-length TTR, which seems to be related to phenotypic variation for the TTR Val30-Met mutation; see, e.g., Suhr et al., J. Internal Medicine 281 (2017), 337-347.

Furthermore, it is known that in various kinds of systemic amyloidosis caused by an amyloid fibril protein deposits of the amyloid fibril protein in tissue and organ co-localize and/or co-aggregate with one or more different amyloid fibril proteins. Indeed, as known in the art and for example published in Higashi et al., Brain Research 1184 (2007) 284-294, tau- and a-synuclein as well as TAR-DNA binding protein 43 (TDP43) are found coexistent in the pathology in the brains of Alzheimer's disease and Dementia with Lewy bodies; see also Lee et al., Trends in Neurosciences 27 (2004), 129-134.

Colocalization of Apolipoprotein AI (apoAI) with amyloid deposits in ATTR amyloidosis has been found as well as in Amyloid A (AA) amyloidosis (AA amyloidosis), immunoglobulin (Ig) lambda light chain amyloidosis (Aλ amyloidosis), Igkappa light chain amyloidosis (Aκ amyloidosis) and Abeta2M amyloidosis; see Sakata et al., J. Histochem. Cytochem. 53 (2005), 237-242. Therefore, artificial *in vitro* generated TTR-V30M aggregates may not reflect the actual structure of ATTR fibrils *in vivo, i.e.* in a patient suffering from ATTR amyloidosis.

In addition, whether the ADCP is significant for the suitability of an anti-TTR antibody for *in vivo* therapeutic application may be questionable and remains to be proven since the antibody used in the model was a humanized mouse monoclonal antibody raised against an artificial antigenic peptide comprising a cryptotope within the TTR sequence and hitherto has been characterized *in vitro* only; see Higaki et al. Amyloid 23 (2016), 86-97.

In contrast, the PDAX model of the present invention employs human patient-derived tissue and fibrils thereof, respectively, and has been validated with a conformation-specific human-derived monoclonal antibody NI-301.37F1 against TTR aggregates, which has been demonstrated to have potential for diagnostic and therapeutic use *in vivo* in transgenic mice expressing exclusively the human V30M-TTR protein and not the mouse TTR protein (FAP mice); see international application WO 2015/092077 A1 and Michalon et al., Orphanet Journal of Rare Diseases 10 (2015), Suppl. 1:P39.

Previously, Yoshimura et al., Bioconjugate Chem. 27 (2016), 1532-1539, described an islet amyloid model mice, established by a method of orthotopic implantation of amylin (islet amyloid polypeptide (IAPP)) aggregates, and intended for studying the development of amylin-imaging probes, in particular ^{99m}Tc-labeled pyridyl benzofuran derivatives. In particular, orthotopic amylin aggregates were prepared *in vitro* by diluting the peptide solution and implanted into in BALB/c-nu/nu mice (8 to 10 weeks old, male) with a surgical method; see Yoshimura *et al.* (2016), *supra,* in section "Orthotopic Amylin Aggregates for Implantation", at page 1537, left column.

However, while in principle the method of implantation could be used for generating a PDAX animal model in accordance with the present invention, the islet amyloid model mice of Yoshimura *et al.* does represent an appropriate animal model for the purposes of the present invention and may not be suitable for the intended purpose. This is because, similar as the *in vivo* model of Higaki *et al.* (2018), *supra,* for ATTR the amylin aggregates prepared *in vitro* cannot be expected to (entirely) reflect the *in vivo* structure of amylin. For example, an association was observed between phosphorylated TDP43 (pTDP43) and IAPP, which was strong and significant particularly in subjects with DM and high extent of IAPP in pancreas; see Leino et al., Journal of Alzheimer's Disease 59 (2017), 43-56.

Hence, one reason for the failure of most approaches so far to provide antibodies in the therapy of amyloidosis may be due to the fact that hitherto antibody candidates have been screened with recombinant amyloid proteins and fibrils thereof generated *in vitro,* which however does not take into account the actual environment and 3-dimensional architecture of the amyloid fibrils in the diseased tissue or organ, which in addition may involve other amyloid fibril forming proteins as well.

In a preferred embodiment, the ATTR fibrils to be implanted are isolated from cardiac tissue, kidney tissue, liver tissue, gastro-intestinal tissue, skin tissue, muscle tissue, tongue tissue, fat tissue, salivary gland tissue, lymph node tissue, brain tissue, pancreatic tissue or any ATTR amyloidoma. In a preferred embodiment, the amyloid fibrils are isolated from cardiac tissue. Methods of isolating amyloid fibrils from tissue have been described before, *e.g.,* in Tennent, Methods Enzymol. 309 (1999), 26-47. Especially, in accordance with the present invention, the amyloid fibrils are isolated using a detergent-free protocol in order to preserve the fibrils' native conformation. The efficiency of the isolation can be verified by standard techniques, like semi-native SDS-PAGE followed by Western blot, known to the person skilled in the art. As described in Example 1 and shown in Fig. 1, amyloid transthyretin (ATTR) fibrils may be efficiently isolated from *post mortem,* frozen cardiac tissue

In accordance with the present invention, the amyloid fibrils are subcutaneously or subcapsularly implanted, implanted in the kidneys, the peritoneum, the muscles, the brain, the ventricles, the nerves, the eyes, the tongue, or the heart. As shown in the Examples, amyloid fibrils are conveniently subcutaneously injected in the tight of mice where they form deposits that are accessible to drug treatments and to further analyses in skin biopsies. Specifically, Example 2 shows that following histological analyses, the deposited amyloid fibrils in skin biopsies of the model according to the present invention show the characteristic features of ATTR amyloids in human biopsies, *i.e.* were Congo red positive, and stained strongly by IHC with the human TTR antibody Dako A0002 (Fig. 2A). Accordingly, in a preferred embodiment the amyloid fibrils are subcutaneously implanted.

Common model animals that are suitable for the present invention are mice, or rats, *i.e.* rodents. In a preferred embodiment of the invention, the animal is a mouse. The mouse as model organism in the purpose of the present invention advantageously is convenient and cheap to house, and has a short generation time, *i.e.* many animals may be produced in a relatively short period of time.

Although at least the mouse genome is relatively easy to manipulate, the establishment of transgenic mouse lines or other animals is always associated with costs and rather time-consuming. Accordingly, it is highly advantageous that for the PDAX model according to the present invention wild-type animals may be used. Therefore, in accordance with the present invention, the animal is non-transgenic for the amyloid fibril protein, and in one embodiment of the present invention, the animal is non-transgenic. Of course, if appropriate also transgenic animals may be used, *e.g.* to better reflect the actual patient's situation. For example, it is conceivable that in order to model a patient suffering from a disease related to an immune deficiency in addition to an amyloidosis, the amyloid fibrils are implanted in immunodeficient mice. This allows the analysis of anti-amyloid drugs in the complex patient system with regard to additional diseases, for example, immune deficiencies or other diseases for which transgenic animal models are available.

In a further aspect, the present invention relates to a method of determining and/or obtaining an anti-amyloid drug suitable for the treatment of an amyloidosis or amyloid-related disease. The method comprises administering the drug, which comprises an anti-amyloid fibril protein antibody, to the model of the present invention and determining amyloid fibrils in the model, wherein the accelerated elimination or reduction of the amyloid fibrils upon administration of the drug compared to a control is indicative for the suitability for the anti-amyloid drug, and preferably the accelerated elimination or reduction is observed in a dose-dependent manner. As illustrated in the Examples a chimeric version of the otherwise fully human antibody can be used for testing while the drug eventually manufactured will be the parent fully human antibody.

The person skilled in the art is aware of various methods of determining amyloid fibrils. As shown in detail in Examples 2 to 5, amyloid fibrils may be analyzed in tissue sections by Congo red staining, thioflavine-S staining or immunohistochemistry with an appropriate antibody. In order to determine whether a drug treatment results in accelerated elimination or reduction of the amyloid fibrils compared to a control, the amyloid fibrils are then quantified using suitable devices and processes, like automated microscopy and image analysis processes. Furthermore, as mentioned in Example 4 and 5, ATTR fibrils undergo spontaneous fibril elimination process in the model. Accordingly, acceleration of fibril elimination or reduction upon treatment with the drug to be assessed is the output for its suitability as an anti-amyloid drug. Accordingly, the method preferably comprises collecting tissue biopsies at a first and second time point after administration, and analysis and quantification of amyloid fibrils including immunohistochemistry. In a further preferred embodiment, the amount of amyloid fibrils is expressed as percentage of the implant tissue area, and the amyloid staining area covering the implant tissue area in the group treated with the drug is significantly lower than in the control group.

In a preferred embodiment, the first time point is 3-10 hours, more preferably 5-8 hours, most preferably 6 hours after administration of the drug or variant thereof and the second time point is 48-156 hours, more preferably 72-120 hours, most preferably 96 hours after administration of the drug or variant thereof.

In principle, all methods for diagnosing amyloidoses in patients' biopsies available may be applied in the method of the present invention in order to determine/analyze the amyloid fibril graft of the model organism.

As shown in Examples 3-5, an ATTR-specific antibody has been shown to specifically bind the implanted amyloid fibrils and to accelerate their elimination. Accordingly, in accordance with the method of the present invention, the drug comprises an anti-amyloid fibril protein antibody. In a further embodiment, the drug is an antibody and the control is a corresponding isotype antibody. Suitable antibody candidates are known from the prior art, *e.g.* anti-transthyretin (TTR) antibodies are disclosed in WO 2015/092077 A1, WO 2014/124334 A2, WO 2018/007923 A3, WO 2016/120810 A1, US 2017/0058023 A1 and US 9,879,080 B2. Of course, it is envisioned within the present invention that especially novel anti-amyloid fibril protein antibodies may be applied to the method of the present invention in order to be assessed for their suitability as anti-amyloid drugs.

As mentioned, most approaches so far to provide drugs in the therapy of amyloidosis employ screening of lead candidate compound with recombinant amyloid proteins and fibrils thereof generated *in vitro,* which however does not take into account the actual environment and 3-dimensional architecture of the amyloid fibrils in the diseased tissue or organ, which in addition may involve other amyloid fibril forming proteins as well.

In contrast, the PDAX animal model of the present invention enables obtaining and selection of anti-amyloid fibril protein antibodies which can be reasonably expected to be as specific as to selectively bind to the target amyloid protein in the patient to be treated at the desired and necessary location of the for example toxic amyloid deposits.

In a particularly preferred embodiment, the candidate antibody is a human antibody or a human chimeric antibody and the constant domain is a rodent constant domain from the same species as the animal employed in the model.

The person skilled in the art is aware of various routes to administer drugs to model animals. Therefore, in one embodiment of the method of the present invention the drug is administered intravenously, intraperitoneally, subcutaneously or orally. For example, the drug is intravenously injection in the tail vein of a mouse having received an implant of isolated amyloid fibrils as shown in Examples 3 and 4.

Administration routes can be taken from corresponding literature known to the person skilled in the art.

In another aspect, the present invention relates to a method for characterization, validation, development and/or quality control of an anti-amyloid drug suitable for the treatment of an amyloidosis or amyloid-related disease. For example, an anti-amyloid drug is characterized in dose-response studies, target engagement experiments (drug binding to target), or is characterized for the treatment mode-of-action, e.g. local activation of a specific immune response by the drug at the fibril graft site, or for the PK/PD relationship, e.g., dose-response as a function of the drug exposure levels. Thereby, the information about the drug that has been subjected to the method of determining and/or obtaining an anti-amyloid drug of the present invention as described, *supra,* is communicated to a client, contracting party or cooperation partner. Furthermore, the drug that has been determined to be a suitable anti-amyloid drug may be selected.

The present invention further relates to the use of the PDAX model for drug characterization, quality control and/or development, pre- and/or co-clinical trials or selecting or validating a drug in the manufacture of a medicament for the treatment of an amyloidosis or an amyloid-related disease. The person skilled in the art is well aware of how to use the model of the present invention in order to characterize a drug, for example in dose-response studies, or target engagement experiments (drug binding to target). Furthermore, in accordance with the present invention, the model is used for characterization of a drug's treatment mode-of-action, e.g. local activation of a specific immune response by the drug at the fibril graft site, or the PK/PD relationship, e.g. dose-response as a function of the drug exposure levels.

In this context, it is also conceivable that the PDAX model is used in the context of personalized medicine, meaning that from an individual patient, amyloid fibrils are isolated and individually analyzed for their response to an anti-amyloid drug using the model of the present invention. With such a use of the model of the present invention, it advantageously is possible to determine whether a treatment is effective in a specific patient before even treating the patient and before subjecting him/her to possible adverse effects. Accordingly, use of the model of the present invention enables a more targeted amyloidosis therapy.

In a further aspect, the present invention relates to a method of producing the patient-derived amyloid xenograft (PDAX) rodent model of the present invention. The method comprises the isolation of ATTR fibrils from a tissue biopsy obtained from a patient suffering from an ATTR amyloidosis and implantation of the isolated amyloid fibrils in a rodent , preferably wherein the fibril preparation has a high viscosity with a total protein concentration of the amyloid fibrils of about 0.5 to 5 mg/ml, preferably about 1 to 4 mg/ml and most preferably about 2 ± 0.5 mg/ml, preferably as determined with a bicinchoninic acid (BCA) assay; see, *e.g.* Smith et al., Anal. Biochem. 150 (1985), 76-85.An example of how the model of the present invention is produced is depicted in Example 1. As described, *supra,* the tissue biopsy from which the amyloid fibrils are isolated is cardiac tissue, kidney tissue, liver tissue, gastro-intestinal tissue, skin tissue, muscle tissue, tongue tissue, fat tissue, salivary gland tissue, lymph node tissue, brain tissue, pancreatic tissue or any other tissue having an amyloidoma deposited. The person skilled in the art is aware of different methods of isolating amyloid fibrils, e.g. isolation methods that preserve the native conformation of the fibrils are described in detail in Tennent, Methods Enzymol. 309 (1999), 26-47. As shown in Example 1, the isolated amyloid fibrils are implanted by subcutaneous injection but other implantation forms like subcapsular implantation, implantation in the kidneys, the peritoneum, the muscles, the brain, the ventricles, the nerves, the eyes, the tongue or the heart are conceivable.

### EXAMPLES

The following Examples 1 to 5 help illustrate embodiments of the invention. The patient-derived amyloid xenograft (PDAX) mouse model presented in the following Examples consists in the subcutaneous implantation of patient-derived ATTR fibrils obtained from post-mortem cardiac tissues.

### Example 1: Production of a PDAX model

### Isolation of ATTR fibrils

Frozen human heart samples presenting massive TTR amyloid infiltration were processed for biochemical extraction of amyloid TTR fibrils. A detergent-free protocol was used to preserve as much as possible the conformation of the fibrils (Pras et al., J. Clin. Invest. 47 (1968), 924-33; Tennent, Methods Enzymol. 309 (1999), 26-47). Briefly, the procedure consisted of repeated mechanical homogenization in ice-cold TE buffer (10 mM Tris pH 8.0, 140 mM NaCl, 10 mM EDTA, 0.1% (wt/vol) NaN₃, Protease inhibitor cocktail) to extract and eliminate soluble proteins, followed by repeated mechanical homogenization in ice-cold pure water. This has been described to remove the calcium-dependent binding of SAP protein on amyloid fibrils allowing the suspension of amyloid fibrils in pure water (Pras *et al.* (1968), *supra*)*.* ATTR fibrils were further purified by precipitation with NaCl and EDTA, and concentrated by centrifugation. The total protein concentration was determined with the BCA assay and was adjusted to 2 mg/ml to ensure high viscosity of the fibril preparation. Each fibril preparation was verified by semi-native SDS-PAGE and Western blot with the ATTR fibril-specific antibody NI-301.37F1 (10 nM) using standard procedure as described hereafter. To preserve the aggregates during the analysis process, samples were mixed with loading buffer and loaded directly on the SDS-PAGE gel, omitting the usual heat denaturation step. For each sample, 10.0 µg of total protein were loaded in 4-12% bis-tris gels in MOPS buffer and ran for 40 min at 200V. Proteins were transferred to a nitrocellulose membrane by semi-dry blotting for 60 min at 20V. Membranes were blocked for 1 h in blocking buffer (2% BSA, 0.1% tween-20 in PBS buffer pH 7.4) and incubated overnight at 4°C with NI-301.37F1 antibody diluted at 10 nM in blocking buffer. Detection was performed using HRP-conjugated anti-human IgG antibody in combination with chemiluminescent substrate.

Fig. 1A shows a semi-native SDS-PAGE and Western blot analysis with NI-301.37F1 of tetrameric WT-TTR (100 ng, not detected), misfolded-aggregated WT-TTR (100 ng), and 0.2, 2.0 and 20 µg per lane of ATTR fibril extract. The tissue extraction procedure resulted in a fraction highly enriched in ATTR fibrils, which were detected by NI-301.37F1 on a semi-native SDS-PAGE gel as aggregates of high molecular weight (Fig. 1A, left panel) that were absent in the secondary antibody only control (Fig. 1A, right panel). A similar experiment was performed with ATTR fibrils prepared from post-mortem cardiac tissues obtained from 4 different donors with ATTR amyloidosis (ATTR+), 4 donors without amyloidosis (ATTR-), and one donor with amyloidosis unrelated to ATTR (A+TTR-) (Fig. 1B). NI-301.37F1 detected selectively patient-derived ATTR fibrils in all ATTR cases, including the one with very low amount of amyloid (sample 70), and did not detect the unrelated proteins present in the same tissue fraction prepared from tissues without ATTR amyloidosis.

### Implantation of patient-derived fibrils

WT SKH1 female mice were briefly anesthetized with isofluorane to receive an implantation of 100 µg ATTR fibril extract by subcutaneous injection on the thigh.

### Anti-TTR Antibody

The mouse chimeric antibody ch.NI-301.37F1 was generated the human-derived monoclonal antibody NI-301.37F1 disclosed in the international application WO 2015/092077 A1. The mouse chimeric variant was designed to contain the human variable domains of NI-301.37F1 in murine constant domain backbones. In particular, the amino acid sequences of the variable heavy (VH) and light (VL) chain of human-derived monoclonal antibody NI-301.37F1 are disclosed in WO 2015/092077 A1 in Figure 1 with SEQ ID NO. 10 and 53, respectively, for the VH chain and SEQ ID NO: 12 for the VL chain, while the mouse heavy chain constant domain corresponds to Uniprot entry P01863 and the mouse light chain constant domain corresponds to Uniprot entry P01837. In brief, gene synthesis was used to produce a synthetic heavy chain gene comprising the sequence coding for the human variable heavy chain of NI-301.37F1 followed by the sequence coding for a murine IgG2a constant heavy chain (cf. sequence mur.37F1 H), and a synthetic light chain gene comprising the sequence coding for the human variable chain of NI-301.37F1 followed by the sequence coding for a murine constant kappa light chain (cf. mur.37F1 L). These 2 genes were then sub-cloned into suitable expression vectors that were used for the transfection of CHO cells. Ch.NI-301.37F1 antibody was purified from the cell culture medium using standard processes as described in WO 2015/092077 A1 including purification of the antibody by chromatography on protein A column.

### Example 2: Characterization of the PDAX model

24 hours after implantation of patient-derived ATTR fibrils, mice were sacrificed, skin tissues including fibril implants were collected, fixed, embedded (PPFA), and cut for histological analysis with Congo red and IHC with the human TTR antibody Dako A0002 and the macrophage markers CD68, F4/80 and Iba1. Congo red staining was performed with Putchler's modifications. In brief, tissue sections were stained with Hemalum, destained in water, incubated successively in solution I (80% EtOH, 30 g/l NaCl, 0.01% NaOH) and solution II (80% EtOH, 30 g/l NaCl, 5 g/l Congo red, 0.01% NaOH) for 30 min each at room temperature (RT), cleared in 100% EtOH with and without 0.01% NaOH, and mounted. Sections were imaged with a 20x objective in bright field and polarization modes. Immunostaining was performed according to standard procedures with quenching of endogenous peroxidase activity with 3% H₂O₂ in methanol for 20 min at RT, incubation in blocking buffer (PBS + 5% serum (horse/goat) + 4% BSA) for 1 hour at RT, followed by incubation with primary antibody overnight at 4°C. Detection was performed using appropriate secondary antibodies (all from Jackson Immuno-research; 1:400) in combination with the Vectastain ABC kit (Vector Laboratories) and diaminobenzidine (Dako). TTR staining was performed with the TTR antibody Dako A0002 at 1:500 dilution in combination with the biotin-conjugated goat anti-rabbit IgG antibody at 1:400 dilution; CD68 antibody was used at 1:400 dilution, the F4/80 antibody at 1:200 dilution, and the IBA1 antibody at 1:750 dilution in combination with, the HRP-conjugated biotin donkey anti rabbit IgG, respectively, at 1:400 dilution in PBS.

The amyloid fibril implants were easily recognizable from the neighboring skin tissue by their shape and appearance, clearly different from the structured and complex morphology of the skin. As shown in Fig. 2A amyloid fibril implants were positive for Congo red staining and TTR IHC. The fibril implants were infiltrated with small cells which stained negative for the murine macrophage markers CD68, F4/80 and Iba1. Instead, as shown in Fig. 2B, the cells infiltrating the amyloid fibril implants stained positive for the monocyte marker CD11b (also named integrin alpha M), indicating that these cells are most certainly neutrophils, which are like macrophages able to do antibody-mediated phagocytosis. Neutrophils are the most abundant immune cell type in the blood. Fibril grafting by s.c. injection creates a microlesion with rupture of local blood capillaries and release of neutrophils which encapsulate the fibril graft, invade it and eliminate it completely in 6 to 8 days. As shown below in Example 4, this process is accelerated in presence of ATTR antibody NI-301.37F1 indicating that the antibody is able to activate the immune system *in vivo.*

### Example 3: In vivo compound binding to ATTR fibrils in the PDAX model

In order to characterize the PDAX model, the *in vivo* binding of an ATTR-specific antibody to implanted patient-derived ATTR fibrils has been analyzed. WT SKH1 female mice received under brief gas anaesthesia an implantation of 100 µg patient-derived ATTR fibril by subcutaneous injection on the thigh, followed by administration of fluorescently labelled NI-301.37F1 or isotype antibodies by intravenous injection in the tail vein at 0.05, 0.5, 5.0 or 15 mg/kg. The antibody labelling with Vivotag-680 was performed according to instructions (Perkin Elmer). In brief, the Vivotag-680 ester-reactive dye was dissolved in DMSO and mixed with antibody prepared in carbonate buffer. After 2 hours of incubation in the dark, the labeled antibody was purified by dialysis overnight at 4°C in PBS buffer.

48 hours after grafting, mice were sacrificed, skin biopsies were collected, fixed, embedded (PPFA), and cut for histological analysis. Sections were rehydrated, stained with thioflavine-S, and mounted with Dapi-containing mounting medium. Slide scanning was performed using an automated fluorescent microscope at 20x magnification using Dapi, FITC and Cy5 channels.

Subcutaneous ATTR fibril implants were clearly recognizable from the neighboring skin tissue by their specific shape and appearance, and presented strong thioflavine-S fluorescence indicating presence of fibrils with preserved amyloid conformation (Fig. 3A). Binding of the fluorescently labelled antibody NI-301.37F1 increased in a dose-dependent manner on the fibrils but not on the surrounding tissues (Fig. 3A); Fibril binding was selective for NI-301.37F1 and was not observed with the isotype antibody. NI-301.37F1 fluorescence overlapped with thioflavine-S fluorescence, indicating binding of NI-301.37F1 on the ATTR fibrils. These results indicate that NI-301.37F1 binds in a dose-dependent manner to patient-derived ATTR fibrils implanted in mice.

NI-301.37F1-VT680 fluorescence intensity at the fibril site was quantified by software-based image analysis and served as a proxy for antibody density. Fluorescence intensity increased continuously across the whole dose range tested; more specifically, there was a continued increase in fluorescence intensity indicating that there was no saturation of NI-301.37F1 binding even between the highest doses tested (5 and 15 mg/kg).

Plasma levels for chimeric NI-301.37F1 were determined with a direct TTR ELISA and a calibration curve with known concentration of antibody. In brief, 96 well microplates were coated for 1 hour at 37°C with human wild-type TTR diluted to a concentration of 10 µg/ml in PBS buffer (pH7.4). Non-specific binding sites were blocked for 1 hour at RT with a blocking buffer (2% BSA, 0.1% tween-20 in PBS buffer pH7.4). Plasma samples were diluted 1:500 in duplicates in blocking buffer. Calibration samples were prepared similarly by diluting chimeric NI-301.37F 1 antibody in duplicates in blocking buffer at a concentration range from 5 pM to 5 nM. Diluted plasma samples were quantified in duplicates (total of quadruplicates per sample): Samples were incubated overnight at 4°C, and chimeric NI-301.37F1 was detected with an HRP-conjugated, anti-mouse IgG2a antibody (Jackson Immunoresearch) at 1:4000 dilution in blocking buffer, followed by measurement of HRP activity using standard procedure.

The analysis of NI-301.37F1-VT680 concentration versus fluorescence intensity on ATTR fibrils revealed a linear correlation across the dose range of 0.5-15 mg/kg. This linear correlation indicates absence of saturation of the target with antibody doses up to 15 mg/kg (Fig. 3B). The results for the dose group 0.05 mg/kg were excluded from the correlation analysis, because the antibody concentrations may have been underestimated due to matrix interference effects at very low concentrations.

### Example 4: In vivo ATTR fibril clearance

Patient-derived ATTR fibrils were implanted in WT mice by subcutaneous injection, followed by administration of mouse chimeric NI-301.37F1 variant (ch.NI-301.37F1) or corresponding isotype antibody at 5.0 mg/kg i.v. Mice were sacrificed 6 or 96 hours later, and skin biopsies were collected for histological analysis. ATTR fibrils were detected by immunohistochemistry using the commercial human TTR antibody Dako A0002. Quantification was performed using automated microscopy and image analysis processes, and the amount of ATTR fibrils was expressed as percentage of the implant tissue area.

Subcutaneous ATTR fibril implants were clearly recognizable from neighboring skin tissue by their specific shape and appearance. 6 hours after implantation and treatment administration, ATTR fibril implants were fully stained for TTR by IHC, with TTR staining covering on average 60 to 70% of the implant tissue area in both treatment groups (Fig. 4). 96 hours after fibril implantation, TTR staining area covered only 12% of the implant tissue area in the group treated with ch.NI-301.37F1, and 39% in the isotype group (Fig. 4). The difference between the two groups was statistically significant with p<0.0001. These results demonstrate that ATTR fibril elimination was accelerated upon treatment with ch.NI-301.37F1.

Similar experiments were conducted based on Congo red staining and thioflavine-S staining which further confirmed that treatment with ch.NI-301.37F1 accelerated ATTR fibril elimination compared to treatment with the isotype antibody.

To further characterize the activity of NI-301.37F1 *in vivo*, ATTR fibril-grafted mice received a single administration of ch.NI-301.37F1 at 0.05, 0.5, 5.0 and 50 mg/kg i.v., or isotype at 50 mg/kg. One mouse from each group was sacrificed 6 hours after drug administration for reference (t0); all other mice were sacrificed after 96 hours, and skin biopsies were collected for histological analysis as described above.

ATTR fibrils covered 86% of the graft area at t0 and decreased to 58% 4 days (96 hours) later in isotype treated mice (Fig. 5), reflecting the spontaneous fibril elimination process in this model. Fibril elimination was accelerated upon treatment with ch.NI-301.37F1 in a dose-dependent manner. Treatment effect was statistically significant at doses of 0.5 mg/kg and above (Fig. 5), but not at the very low dose of 0.05 mg/kg due to limited effect size and low number of mice per dose group. Complete fibril elimination was obtained with both 5.0 and 50 mg/kg doses.

These data confirm the activity of NI-301.37F1 *in vivo* and indicate that activation of the immune system for the elimination of ATTR fibrils occurs already at low doses.

### Example 5: Characterization of the PDAX model upon antibody treatment

*In vivo* clearance of ATTR fibrils in the PDAX model as shown in Example 4 was further characterized by staining for the myeloid cell markers IBA1, DC11B and LY6G.

IBA1 also known as allograft inflammatory factor 1 (AIF1) is a marker for macrophages as well as activated neutrophils. ATTR graft tissues were stained for IBA1 expression by IHC. Cells present within and around the ATTR graft tissues did not present any IBA1 expression 6 hours after grafting (Fig. 6A). Similarly, IBA1 expression 96 hours after grafting was low or absent and detected only in a small fraction of the cells within the ATTR graft area.

Cluster of differentiation molecule 11B (CD11B) is also known under the names integrin alpha M (ITGAM), macrophage-1 antigen (Mac-1) or complement receptor 3 (CR3), and is expressed by monocytes and macrophages but also granulocytes and natural killer cells. ATTR graft tissues stained for CD11B expression by IHC presented CD11B positive cells within and adjacent to the graft tissues 6 hours after grafting (Fig. 6B). At this timepoint, CD11B positive cells were typically small and amoeboid, with a subset of cells presenting extended processes. 96 hours after grafting we observed a large increase in CD11B positive cells which had infiltrated the entire ATTR graft in both control antibody as well as chNI-301.37F1 treated mice.

LY6G is a GPI-anchored protein and, together with LY6C, a component of the myeloid differentiation antigen GR-1. LY6G is predominantly present on peripheral neutrophils. ATTR graft sections stained for LY6G expression presented with LY6G positive cells within and around the ATTR grafts 6 hours after grafting independent of treatment (Fig. 6C). In contrast, no LY6G expressing cells were detected 96 hours after grafting.

## Claims

1. A patient-derived amyloid xenograft (PDAX) non-human animal model, wherein the animal is **characterized by** an implant of amyloid fibrils obtained from the tissue or organ of a patient suffering from an amyloidosis, wherein the amyloid and amyloid fibrils, respectively, comprise amyloid transthyretin (ATTR), and wherein the amyloid fibrils are subcutaneously or subcapsularly implanted or implanted in the kidney, the peritoneum, the muscles, the brain, the ventricles, the nerves, the eyes, the tongue, or the heart, wherein said animal is a rodent and non-transgenic for the amyloid fibril protein.

2. The model of claim 1, wherein the rodent is a mouse or a rat.

3. The model of claim 1 or 2, wherein the animal is non-transgenic.

4. The model of any one of claims 1 to 3, wherein the model comprises an implant of amyloid fibrils obtained from the tissue or organ of a human patient suffering from amyloidosis, wherein the amyloid fibrils comprise amyloid transthyretin (ATTR), and wherein the amyloid fibrils are subcutaneously implanted.

5. A method of determining and/or obtaining an anti-amyloid drug suitable for the treatment of an amyloidosis or amyloid-related disease comprising
(a) administering the drug to the model of any one of claims 1 to 4; and
(b) determining amyloid fibrils in the model, wherein the accelerated elimination or reduction of the amyloid fibrils upon administration of the drug compared to a control is indicative for the suitability for the anti-amyloid drug, wherein the drug comprises an anti-amyloid fibril protein antibody.

6. The method of claim 5, wherein the method comprises
(i) collecting tissue biopsies at a first and second time point after administration, and
(ii) analysis and quantification of amyloid fibrils including immunohistochemistry, preferably wherein the amount of amyloid fibrils is expressed as percentage of the implant tissue area, and the amyloid staining area covering the implant tissue area in the group treated with the drug is significantly lower than in the control group.

7. The method of claim 5 or 6, wherein elimination or reduction of the amyloid fibrils is observed in a dose dependent manner.

8. The method of any one of claims 5 to 7, wherein the control is a corresponding isotype antibody.

9. The method of any one of claims 5 to 8, wherein the antibody is a human antibody or a human chimeric antibody comprising a rodent constant domain from the same species as the animal employed in the model.

10. The method of any one of claims 5 to 9, wherein the drug is administered intravenously, intraperitoneally, subcutaneously or orally.

11. A method for characterization, validation, development and/or quality control of an anti-amyloid drug suitable for the treatment of an amyloidosis or amyloid-related disease comprising
(i) subjecting the drug to the method of any one of claims 5 to 10;
(ii) communicating the information obtained in (i) to a client, contracting party or cooperation partner and/or selecting the drug that has been determined to be a suitable anti-amyloid drug.

12. Use of the model of any one of claims 1 to 4 for drug characterization, quality control and/or development, pre- and/or co-clinical trials or selecting or validating a drug in the manufacture of a medicament for the treatment of an amyloidosis or an amyloid-related disease.

13. A method of producing a patient-derived amyloid xenograft (PDAX) non-human animal model of any one of claims 1 to 4 comprising
(i) isolation of amyloid fibrils from a tissue biopsy obtained from a patient suffering from an amyloidosis, and
(ii) implantation of the isolated amyloid fibrils in a rodent, preferably wherein the total protein concentration of the amyloid fibrils is about 0.5 to 5 mg/ml, preferably about 1 to 4 mg/ml and most preferably about 2 ± 0.5 mg/ml.

## Patentansprüche

1. Vom Patienten stammendes Amyloid-Xenotransplantat (PDAX) nicht-menschliches Tiermodell, wobei das Tier durch ein Implantat von Amyloidfibrillen gekennzeichnet ist, die aus dem Gewebe oder Organ eines an einer Amyloidose leidenden Patienten gewonnen wurden, wobei das Amyloid bzw. die Amyloidfibrillen Amyloid-Transthyretin (ATTR) umfassen, und wobei die Amyloidfibrillen subkutan oder subkapsulär implantiert oder in die Niere, das Peritoneum, die Muskeln, das Gehirn, die Ventrikel, die Nerven, die Augen, die Zunge oder das Herz implantiert werden, wobei das Tier ein Nagetier und nicht-transgen für das Amyloidfibrillenprotein ist.

2. Modell nach Anspruch 1, wobei das Nagetier eine Maus oder eine Ratte ist.

3. Modell nach Anspruch 1 oder 2, wobei das Tier nicht-transgen ist.

4. Modell nach einem der Ansprüche 1 bis 3, wobei das Modell ein Implantat von Amyloidfibrillen umfasst, die aus dem Gewebe oder Organ eines an Amyloidose leidenden menschlichen Patienten gewonnen wurden, wobei die Amyloidfibrillen Amyloid-Transthyretin (ATTR) umfassen und wobei die Amyloidfibrillen subkutan implantiert werden.

5. Verfahren zur Bestimmung und/oder Gewinnung eines Anti-Amyloid-Arzneimittels, das für die Behandlung einer Amyloidose oder einer mit Amyloid zusammenhängenden Krankheit geeignet ist, umfassend
(a) Verabreichung des Arzneimittels an das Modell nach einem der Ansprüche 1 bis 4; und
(b) Bestimmung von Amyloidfibrillen in dem Modell, wobei die beschleunigte Eliminierung oder Verringerung der Amyloidfibrillen nach Verabreichung des Arzneimittels im Vergleich zu einer Kontrolle ein Hinweis auf die Eignung für das Anti-Amyloid-Arzneimittel ist, wobei das Arzneimittel einen Anti-Amyloidfibrillen-Protein-Antikörper umfasst.

6. Verfahren nach Anspruch 5, wobei das Verfahren umfasst
(i) Entnahme von Gewebebiopsien zu einem ersten und einem zweiten Zeitpunkt nach der Verabreichung, und
(ii) Analyse und Quantifizierung von Amyloidfibrillen einschließlich Immunhistochemie, wobei vorzugsweise die Menge der Amyloidfibrillen als Prozentsatz der Implantatgewebefläche ausgedrückt wird und die Amyloidfärbefläche, die die Implantatgewebefläche in der mit dem Arzneimittel behandelten Gruppe bedeckt, signifikant niedriger ist als in der Kontrollgruppe.

7. Verfahren nach Anspruch 5 oder 6, wobei die Eliminierung oder Verringerung der Amyloidfibrillen in einer dosisabhängigen Weise beobachtet wird.

8. Verfahren nach einem der Ansprüche 5 bis 7, wobei die Kontrolle ein entsprechender Isotyp-Antikörper ist.

9. Verfahren nach einem der Ansprüche 5 bis 8, wobei der Antikörper ein menschlicher Antikörper oder ein menschlicher chimärer Antikörper ist, der eine konstante Domäne eines Nagetiers aus der gleichen Spezies wie das im Modell verwendete Tier umfasst.

10. Verfahren nach einem der Ansprüche 5 bis 9, wobei das Arzneimittel intravenös, intraperitoneal, subkutan oder oral verabreicht wird.

11. Verfahren zur Charakterisierung, Validierung, Entwicklung und/oder Qualitätskontrolle eines Anti-Amyloid-Arzneimittel, das für die Behandlung einer Amyloidose oder einer mit Amyloid zusammenhängenden Krankheit geeignet ist, umfassend
(i) Unterziehen des Arzneimittels dem Verfahren nach einem der Ansprüche 5 bis 10;
(ii) Weitergabe der unter (i) gewonnenen Informationen an einen Kunden, Vertragspartner oder Kooperationspartner und/oder Auswahl des Arzneimittels, das sich als geeignetes Anti-Amyloid-Arzneimittel erwiesen hat.

12. Verwendung des Modells nach einem der Ansprüche 1 bis 4 für die Arzneimittelcharakterisierung, Qualitätskontrolle und/oder Entwicklung, prä- und/oder co-klinische Studien oder die Auswahl oder Validierung eines Wirkstoffs bei der Herstellung eines Arzneimittels zur Behandlung einer Amyloidose oder einer mit Amyloid zusammenhängenden Krankheit.

13. Verfahren zur Herstellung eines vom Patienten stammendes Amyloid-Xenotransplantat (PDAX) nicht-menschlichen Tiermodells nach einem der Ansprüche 1 bis 4, umfassend
(i) Isolierung von Amyloidfibrillen aus einer Gewebebiopsie eines Patienten, der an einer Amyloidose leidet, und
(ii) Implantation der isolierten Amyloidfibrillen in ein Nagetier, wobei vorzugsweise die Gesamtproteinkonzentration der Amyloidfibrillen etwa 0,5 bis 5 mg/ml, vorzugsweise etwa 1 bis 4 mg/ml und am meisten bevorzugt etwa 2 ± 0,5 mg/ml beträgt.

## Revendications

1. Modèle animal non humain de xénogreffe amyloïde (PDAX) dérivée d'un patient, dans lequel l'animal est **caractérisé par** un implant de fibrilles amyloïdes obtenues à partir du tissu ou de l'organe d'un patient souffrant d'une amyloïdose, dans lequel l'amyloïde et les fibrilles amyloïdes, respectivement, comprennent de la transthyrétine amyloïde (ATTR), et dans lequel les fibrilles amyloïdes sont implantées par voie sous-cutanée ou implantées par voie sous-capsulaire dans le rein, le péritoine, les muscles, le cerveau, les ventricules, les nerfs, les yeux, la langue ou le coeur, dans lequel ledit animal est un rongeur et non transgénique pour la protéine de fibrille amyloïde.

2. Modèle selon la revendication 1, dans lequel le rongeur est une souris ou un rat.

3. Modèle selon la revendication 1 ou 2, dans lequel l'animal n'est pas transgénique.

4. Modèle selon l'une quelconque des revendications 1 à 3, dans lequel le modèle comprend un implant de fibrilles amyloïdes obtenues à partir du tissu ou de l'organe d'un patient humain souffrant d'amyloïdose, dans lequel les fibrilles amyloïdes comprennent de la transthyrétine amyloïde (ATTR), et dans lequel les fibrilles amyloïdes sont implantées par voie sous-cutanée.

5. Procédé de détermination et/ou d'obtention d'un médicament anti-amyloïde approprié pour le traitement d'une amyloïdose ou d'une maladie liée à l'amyloïde, comprenant les étapes consistant à
(a) administrer le médicament au modèle selon l'une quelconque des revendications 1 à 4 ; et
(b) déterminer les fibrilles amyloïdes dans le modèle, dans lequel l'élimination ou la réduction accélérée des fibrilles amyloïdes lors de l'administration du médicament par rapport à un témoin est indicative de l'adéquation au médicament anti-amyloïde, dans lequel le médicament comprend un anticorps de protéine de fibrille anti-amyloïde.

6. Procédé selon la revendication 5, dans lequel le procédé comprend les étapes consistant à :
(i) collecter des biopsies tissulaires à un premier et à un second temps après l'administration, et
(ii) analyser et quantifier les fibrilles amyloïdes, incluant l'immunohistochimie, de préférence dans lequel la quantité de fibrilles amyloïdes est exprimée en pourcentage de la surface tissulaire de l'implant, et la zone de coloration d'amyloïde couvrant la surface tissulaire de l'implant dans le groupe traité avec le médicament est significativement plus faible que dans le groupe témoin.

7. Procédé selon la revendication 5 ou 6, dans lequel l'élimination ou la réduction des fibrilles amyloïdes est observée d'une manière dépendant de la dose.

8. Procédé selon l'une quelconque des revendications 5 à 7, dans lequel le témoin est un anticorps isotypique correspondant.

9. Procédé selon l'une quelconque des revendications 5 à 8, dans lequel l'anticorps est un anticorps humain ou un anticorps chimérique humain comprenant un domaine constant de rongeur provenant de la même espèce que l'animal utilisé dans le modèle.

10. Procédé selon l'une quelconque des revendications 5 à 9, dans lequel le médicament est administré par voie intraveineuse, intrapéritonéale, sous-cutanée ou orale.

11. Procédé pour la caractérisation, la validation, le développement et/ou le contrôle de la qualité d'un médicament anti-amyloïde approprié pour le traitement d'une amyloïdose ou d'une maladie liée à l'amyloïde, comprenant les étapes consistant à
(i) soumettre le médicament au procédé selon l'une quelconque des revendications 5 à 10 ;
(ii) communiquer les informations obtenues en (i) à un client, à une partie contractante ou à un partenaire de coopération et/ou sélectionner le médicament qui a été déterminé comme étant un médicament anti-amyloïde approprié.

12. Utilisation du modèle selon l'une quelconque des revendications 1 à 4 pour des essais pré- et/ou co-cliniques de caractérisation, de contrôle qualité et/ou de développement d'un médicament, ou la sélection ou la validation d'un médicament dans la fabrication d'un médicament pour le traitement d'une amyloïdose ou d'une maladie liée à l'amyloïde.

13. Procédé de production d'un modèle animal non humain de xénogreffe amyloïde (PDAX) dérivé d'un patient selon l'une quelconque des revendications 1 à 4, comprenant les étapes consistant à
(i) isoler des fibrilles amyloïdes à partir d'une biopsie tissulaire obtenue auprès d'un patient souffrant d'amyloïdose, et
(ii) implanter les fibrilles amyloïdes isolées chez un rongeur, de préférence dans lequel la concentration en protéines totales des fibrilles amyloïdes est d'environ 0,5 à 5 mg/ml, de préférence d'environ 1 à 4 mg/ml, et de manière tout à fait préférée d'environ 2 ± 0,5 mg/ml.
